# EUROPEAN PATENT APPLICATION

(11) **EP 2 295 962 A1**
(43) Date of publication of application: **16.03.2011**
(21) Application number: 09762265.8
(22) Date of filing: 10.06.2009
(51) Int. Cl.: G01N 30/88, G01N 27/447, G01N 27/62, G01N 30/06, G01N 30/26, G01N 30/72

(54) **METHOD FOR PREPARATION OF SUGAR CHAIN SAMPLE, SUGAR CHAIN SAMPLE, AND METHOD FOR ANALYSIS OF SUGAR CHAIN**

(30) Priority: 12.06.2008 JP 2008154649
(71) Applicant: Sumitomo Bakelite Co., Ltd., Shinagawa-ku Tokyo 140-0002 (JP)
(72) Inventor: SHIMAOKA, Hideyuki, Tokyo 140-0002 (JP); ABE, Midori, Tokyo 140-0002 (JP)
(74) Representative: Müller, Christian Stefan Gerd
(86) International application number: PCT/JP2009/002611
(87) International publication number: WO 2009/150834

(57) **Abstract**

A method of preparing a sugar chain sample, for reducing unreacted labeling reagent in a sample solution containing a labeled sugar chain, includes (process 1) a process of bringing the sample solution containing the labeled sugar chain into contact with monolithic silica, so as to allow the monolithic silica to adsorb a sugar chain component; (process 2) a process of washing the monolithic silica with a washing liquid; and (process 3) a process of bringing the monolithic silica into contact with an eluant, so as to elute the adsorbed sugar chain.

## Description

### TECHNICAL FIELD

The present invention relates to a method of purifying a labeled sugar chain.

### BACKGROUND ART

Sugar chain is a general term for molecules composed of monosaccharides such as glucose, galactose, mannose, fucose, xylose, N-acetylglucosamine, N-acetylgalactosamie and sialic acid, and derivatives of them, bound via glycoside bonds to give a chain form.
The sugar chain is diversified over a broad range, and takes part in various functions owned by naturally-occurring lives. The sugar chain often exist *in vivo* while being bound with protein or lipid, to give a form of glycoconjugate which is one of important constituents of living bodies. It has increasingly been revealed that the sugar chain in living bodies deeply correlates to regulation of inter-cellular information transfer, functions of proteins, and interaction with proteins.

For example, biopolymers having sugar chain include proteoglycans in plant cell walls contributive to stabilization of cells, glycolipids affective to differentiation, proliferation, adhesion, migration of cells, and glycoproteins contributive to inter-cellular interaction and cell recognition. Mechanisms by which the sugar chain of biopolymers controls biological reactions in a sophisticated and precise manner, while mutually substituting, assisting, amplifying, regulating, or inhibiting the functions, has gradually been made clear. If correlations of the sugar chain with differentiation and proliferation of cells, cell adhesion, immunity, and cancerous change of cells are made clear, the sugar chain engineering is expected to explore new applications, while being closely tied with medical science, cell engineering or organ engineering.

For the purpose of early detection of disease and retention of quality of life (QOL), a biomarker, based on which onset of disease may be prevented or transition of disease may be diagnosed, is necessary. From analytical study of gene-disrupted mouse lacking sugar transferase responsible for biosynthesis of sugar chain, it has been revealed that the sugar chain is indispensable for functional retention of various tissues and organs (Non-Patent Documents 1, 2). It has also been known that abnormal modification of the sugar chain may be causative of various diseases (Non-Patent Document 3). Since the structure of the sugar chain may distinctively be vary depending on cancerous changes of cells and various diseases, the sugar change is expected to be utilized as a biomarker for investigating transition of diseases.

Since the sugar chain per se shows no fluorescence or ultraviolet absorption, so that the sugar chain is often modified (labeled) prior to analysis. For analysis by HPLC or HPLC-MS, the sugar chain is generally labeled with phosphorescence, typically by modifying it into 2-aminobenzamide derivative (2AB modification), or into 2-aminopyridine derivative (PA modification) (Non-Patent Document 4). Also for analysis by MALDI-TOF MS, the sugar chain is labeled so as to improve sensitivity of measurement (Non-Patent Documents 5, 6).

In the process of labeling, the sugar chain is often reacted with an excessive amount of labeling reagent, for the purpose of improving efficiency of labeling. Since an excessive amount of labeling reagent which resides in a sample solution may adversely affect the HPLC measurement or mass analysis, so that it is necessarily removed in advance. The reagent is removed typically by adopting a method of gel filtration (typically by using Sephadex G-15, Non-Patent Document 6), or a method of solid phase extraction (typically by using silica gel, graphite carbon, and so forth). Conventionally, the separation has been carried out typically by using an appropriate container vessel (column, cartridge, etc.) packed with a solid phase material (filler). A method for improving resolution of the column (cartridge) having generally been adopted is either one of increase in packing density of the solid phase material, and reduction in number-average diameter of the filler. However, the former has failed in increasing the packing density up to an expected level, due to variation in geometry and diameter of the filler, whereas the latter has tended to be limited in process speed, due to increased load of pressure to the column or apparatus. In addition, a small-scale column (cartridge) has been more likely to degrade resolution and yield, due to a dead volume of a frit (a component for stopping beads) necessary for holding the filler. A means for solving these problems and enabling efficient purification of the labeled sugar chain has therefore been desired.

[Non-Patent Document 1] Ioffe E., Stanley P., Proc. Natl. Acad. Sci., 91, pp.728-732 (1994)
[Non-Patent Document 2] Metzler M., Gertz A., Sarker M., Schachter H., Schrader J.W., Marth J.D., EMBO J., 13, pp.2056-2065 (1994)
[Non-Patent Document 3] Powell L.D., Paneerselvam K., Vij R., Diaz S., Manzi A., Buist N., Freeze H., Varki A., J. Clin. Invest., 94, pp.1901-1909 (1994)
[Non-Patent Document 4] Shilova N.V., Bovin N.V., Russian Journal of Bioorganic Chemistry, 29, pp.309-324 (2003)
[Non-Patent Document 5] Shinohara Y., Furukawa J. -i., Niikura K., Miura Y., Nishimura S.-I., Anal. Chem., 76, pp.6989-6997 (2004)
[Non-Patent Document 6] Furukawa J. -i., Shinohara Y., Kuramoto H., Miura Y., Shimaoka H., Kurogochi M., Nakano M., Nishimura S.-I., Anal. Chem., 80, pp.1094-1101 (2008)

### DISCLOSURE OF THE INVENTION

It is therefore an object of the present invention to provide a means for efficiently removing an excessive labeling reagent, after labeling of a sugar chain.

The present invention is as follows.
(1) A method of preparing a sugar chain sample for reducing an unreacted labeling reagent in a sample solution containing a labeled sugar chain, which includes:
   (process 1) a process of bringing the sample solution containing the labeled sugar chain into contact with monolithic silica, so as to allow the monolithic silica to adsorb a sugar chain component;
   (process 2) a process of washing the monolithic silica with a washing liquid; and
   (process 3) a process of bringing the monolithic silica into contact with an eluant, so as to elute the adsorbed sugar chain.
(2) The method of preparing a sugar chain sample as described in (1), wherein process 1 includes a process of injecting sample solution into a columnar vessel having monolithic silica packed in the flow path thereof; and a process of allowing the sample solution to pass through the monolithic silica portion based on free fall, suction, pressurization or centrifugation.
(3) The method of preparing a sugar chain sample as described in (1) or (2), wherein, in process 1, the sample solution contains 90% by volume or more of an organic solvent.
(4) The method of preparing a sugar chain sample as described in (1) or (2), wherein, in process 1, the sample solution contains 95% by volume or more of an organic solvent.
(5) The method of preparing a sugar chain sample as described in (3) or (4), wherein the organic solvent is acetonitrile.
(6) The method of preparing a sugar chain sample as described in any one of (1) to (5), wherein, in process 2, the washing liquid is a solution containing an organic solvent and water.
(7) The method of preparing a sugar chain sample as described in (6), wherein, in process 2, the washing liquid is a solution containing 90% by volume or more of the organic solvent and 10% by volume or less of water.
(8) The method of preparing a sugar chain sample as described in (6), wherein, in process 2, the washing liquid contains 95% by volume or more of the organic solvent, and 5% by volume or less of water.
(9) The method of preparing a sugar chain sample as described in any one of (6) to (8), wherein the organic solvent is acetonitrile.
(10) The method of preparing a sugar chain sample as described in any one of (1) to (9), wherein, in process 3, the eluant is a solution containing 10% by volume or more of water.
(11) The method of preparing a sugar chain sample as described in any one of (1) to (9), wherein, in process 3, the eluant is a solution containing 50% by volume or more of water.
(12) The method of preparing a sugar chain sample as described in any one of (1) to (9), wherein, in process 3, the eluant is water.
(13) The method of preparing a sugar chain sample as described in any one of (1) to (12), wherein the labeled sugar chain has a compound having an amino group, hydrazide group, or aminoxy group, bound to the reducing end thereof.
(14) The method of preparing a sugar chain sample as described in any one of (1) to (12), wherein the labeled sugar chain has at least one amino group-containing compound selected from those listed below, bound to the reducing end thereof:
   2-aminopyridine; 2-aminobenzamide; 2-aminoanthranilic acid;
   7-amino-1-naphthol; 3-(acetylamino)-6-aminoacridine;
   9-aminopyrene-1,4,6-trisulfonic acid;
   8-aminonaphtalene-1,3,6-tri-sulfonic acid;
   7--amino-1,3-naphtalenedisulfonic acid: 2-amino-9(10H)-acridone;
   5-aminofluorescein; Dansylethylenediamine;
   7-amino-4-methylcoumarine; and benzylamine.
(15) The method of preparing a sugar chain sample as described in any one of (1) to (12), wherein the labeled sugar chain has at least one hydrazide group-containing compound selected from those listed below, bound to the reducing end thereof:
   2-aminobenzhydrazide; 2-hydrazinobenzoic acid; benzylhydrazine;
   5-Dimethylaminonaphthalene-1-sulfonyl hydrazine (Dansylhydrazine); 2-hydrazinopyridine; 9-fluorenylmethyl carbazate (Fmoc hydrazine);
   4,4-difluoro-5,7-dimethyl-4-bora-3a,4a-diaza-s-indacene-3-propionic acid, hydrazide;
   2-(6,8-difluoro-7-hydroxy-4-methylcoumarin) acetohydrazide;
   7-diethylaminocoumarin-3-carboxylic acid, hydrazide (DCCH);
   phenylhydrazine;1-Naphthaleneacethydrazide and phenylacetic hydrazide.
(16) The method of preparing a sugar chain sample as described in any one of (1) to (12), wherein the labeled sugar chain has at least one aminoxy group-containing compound selected from those listed below, bound to the reducing end thereof:
   N-aminooxyacetyl(tryptophyl)arginine methyl ester;
   O-benzylhydroxylamine; O-phenylhydroxylamine; O-(2,3,4,5,6-pentafluorobenzyl)hydroxylamine; O-(4-nitrobenzyl)hydroxylamine;
   2- aminooxypyridine; 2-aminooxymethylpyridine; 4-[(aminooxyacetyl) amino]benzoic acid methyl ester; 4-[(aminooxyacetyl)amino]benzoic acid ethyl ester; 4-[(aminooxyacetyl)amino]benzoic acid *n*-butyl ester; and aminooxy-biotin.
(17) A sugar chain sample prepared by the method of preparing a sugar chain sample described in any one of (1) to (16).
(18) A method of analyzing a sugar chain analyzing the sugar chain sample, prepared by the method of preparing a sugar chain sample described in any one of (1) to (16), by an analytical means such as HPLC, mass spectrometry, LC-MS, or capillary electrophoresis.

The method of the present invention facilitates removal of an excessive amount of labeling reagent after labeling of a sugar chain, and thereby contributes to improve efficiency of analysis of the sugar chain.

### BRIEF DESCRIPTION OF THE DRAWINGS

FIG. 1 is an HPLC chart of 2AB-modified sugar chain (N-type sugar chain of fetuin) purified by using monolithic silica;
FIG. 2 is an HPLC chart of PA-modified sugar chain (N-type sugar chain of fetuin) purified by using monolithic silica;
FIG. 3 is an enlarged view of FIG. 2; and
FIG. 4 is an HPLC chart of 2AB-modified sugar chain (N-type sugar chain of fetuin) purified by using granular silica gel.

### BEST MODES FOR CARRYING OUT THE INVENTION

The present invention is a method of preparing a sugar chain sample for reducing an unreacted labeling reagent in a sample solution containing a labeled sugar chain, which includes:
(process 1) a process of bringing the sample solution containing the labeled sugar chain into contact with monolithic silica, so as to allow the monolithic silica to adsorb a sugar chain component;
(process 2) a process of washing the monolithic silica with a washing liquid; and
(process 3) a process of bringing the monolithic silica into contact with an eluant, so as to elute the adsorbed sugar chain.
In (process 1) to (process 3), the labeled sugar chain and the unreacted labeling reagent may be separated, the purified labeled sugar chain may be obtained as a prepared sugar chain sample, and the labeled sugar chain may be analyzed by various methods of analysis.

### (Sugar Chain Sample)

The sugar chain sample used in the present invention may be prepared from biological samples such as whole blood, serum, plasma, urine, saliva, cell, tissue, virus, and plant tissue. Alternatively, also those prepared from purified or unpurified glycoproteins may be adoptable. Also a pure sugar chain preliminarily purified elsewhere may be adoptable as the sample. The sugar chain is released from glycoprotein contained in the above-described biological samples, using a means for releasing sugar chain. The means for releasing sugar chain adoptable herein may be exemplified by glycosidase treatment using N-glycosidase or O-glycosidase, hydrazine decomposition, β elimination under alkaline treatment, and so forth. Analysis of N-type sugar chain may preferably be carried out by using N-glycosidase. Alternatively, protease treatment using trypsin, chymotrypsin or the like, may be conducted prior to the glycosidase treatment.

### (Purification of Sugar Chain)

Prior to the labeling of the sugar chain, impurities other than the sugar chain (protein, peptide, nucleic acid, lipid, and so forth) may preferably be removed. Methods of removing impurities adoptable herein include gel filtration, ultrafiltration, dialysis, solid phase extraction based on polarity, purification using ion exchange resin, precipitation based on difference in solubility, and so forth. Purification using a solid phase carrier (for example, BlotGlyco BS-45601S from Sumitomo Bakelite Co., Ltd., see Non-Patent Document 6) capable of selectively binding the sugar chain is adoptable. Alternatively, the sugar chain may be labeled without removing the impurities.

### (Labeling of Sugar Chain)

The sugar chain may be labeled by adding a labeling reagent and allowing them to react, after the sample containing the sugar chain is dried if occasion demands. The labeling reaction is preferably reductive amination by which the sugar chain is labeled with an arbitrary amino compound. The labeling reagent is preferably a substance having a functional group such as amino group, hydrazide group, aminoxy group or the like, which is reactive with the reducing end of the sugar chain.

Substances containing amino group may preferably be selected from the group consisting of the compounds listed below, but are not limited thereto:
2-aminopyridine; 2-aminobenzamide; 2-aminoanthranilic acid;
7-amino-1-naphthol; 3-(acetylamino)-6-aminoacridine;
9-aminopyrene-1,4,6-trisulfonic acid;
8-aminonaphtalene-1,3,6-trisulfonic acid;
7-amino-1,3-naphtalenedisulfonic acid: 2-amino-9(10H)-acridone;
5-aminofluorescein; Dansylethylenediamine;
7-amino-4-methylcoumarine; and benzylamine.
Among these compounds, 2-aminopyridine(PA-modified), 2-aminobenzamide (2AB-modified), 2-aminoanthranilic acid (2AA-modified), and 3-(acetylamino)-6-aminoacridine (AA-Ac-modified), having widely been used as labeling reagents for HPLC analysis, are particularly preferable.
8-Aminonaphtalene-1,3,6-trisulfonic acid (APTS), having widely been used for analysis of sugar chain based on capillary electrophoresis, is particularly preferable.

Substances containing hydrazide group may preferably be selected from the group consisting of the compounds listed below, but are not limited thereto:
2-aminobenzhydrazide; 2-hydrazinobenzoic acid; benzylhydrazine;
5-dimethylaminonaphthalene-1-sulfonyl hydrazine (Dansylhydrazine); 2-hydrazinopyridine; 9-fluorenylmethyl carbazate (Fmoc hydrazine);
4,4-difluoro-5,7-dimethyl-4-bora-3a,4a-diaza-s-indacene-3-propionic acid, hydrazide;
2-(6,8-difluoro-7-hydroxy-4-methylcoumarin) acetohydrazide;
7-diethylaminocoumarin-3-carboxylic acid, hydrazide(DCCH); phenylhydrazine; 1-Naphthaleneacethydrazide; and phenylacetic hydrazide.

Substances containing aminoxy group may preferably be selected from the group consisting of the compounds listed below, but are not limited thereto:
N-aminooxyacetyl(tryptophyl)arginine methyl ester;
O-benzylhydroxylamine; O-phenylhydroxylamine; O-(2,3,4,5,6-pentafluorobenzyl) hydroxylamine; O-(4-nitrobenzyl) hydroxylamine;
2- aminooxypyridine; 2-aminooxymethylpyridine; 4-[(aminooxyacetyl) amino]benzoic acid methyl ester; 4-[(aminooxyacetyl)amino]benzoic acid ethyl ester; 4-[(aminooxyacetyl)amino]benzoic acid *n*-butyl ester; and aminooxy-biotin.
Among these compounds, N-aminooxyacetyl(tryptophyl)arginine methyl ester (aoWR), having been known to express an effect of improving sensitivity in MALDI-TOF MS measurement, is particularly preferable.

While methods of synthesizing the above-described compounds are not specifically limited, the synthesis may follow publicly known methods such as described, for example, in International Publication Pamphlet W02008/018170.
While the method of the present invention is not limited by conditions of the labeling reaction, it is preferable, for example, to add 10-fold equivalence or more of a labeling reagent relative to the sugar chain, and to allow the reaction to proceed under heating in the presence of a reducing agent. In the reaction system, pH is preferably adjusted to acidic to neutral, preferably to the range from 2 to 9, and more preferably from 2 to 8, and still more preferably from 2 to 7. Reaction temperature is preferably adjusted to the range from 4 to 90°C, more preferably from 25 to 90°C, and still more preferably from 40 to 90°C. Reaction time is preferably adjusted to the range from 10 minutes to 24 hours, more preferably from 10 minutes to 8 hours, and still more preferably from 10 minutes to 3 hours. The reducing agent is not always necessarily added, for the case where the labeling is proceeded using hydrazide group-containing compounds, or aminoxy group-containing compound.

In particular for the case where the amino compound is 2-aminobenzamide, pH condition is adjusted to acidic to neutral, preferably to the range from 2 to 9, more preferably from 2 to 8, and still more preferably from 2 to 7. The reaction temperature is 4 to 90°C, preferably 30 to 90°C, and more preferably 40 to 80°C. Concentration of the amino compound is 1 mM to 10 M, preferably 10 mM to 10 M, and more preferably 100 mM to 1 M. Concentration of the reducing agent is 1 mM to 10 M, preferably 10 mM to 10 M, and more preferably 100 mM to 2 M. Reaction time is 10 minutes to 24 hours, preferably 10 minutes to 8 hours, and more preferably 1 hour to 3 hours.

Examples of the reducing agent adoptable herein include sodium cyanoborohydride, methylamine borane, dimethylamine borane, trimethylamine borane, picoline borane, and pyridine borane. Sodium cyanoborohydride may preferably be used, in view of reactivity.

### (Purification of Labeled Sugar Chain)

Since the labeled sugar chain solution prepared by the method described in the above contains the unreacted labeling reagent, so that it is difficult to directly analyze the solution. For this reason, the unreacted labeling reagent is necessarily removed prior to the measurement. For removal of the reagent, it is often to use a device composed of a column or cartridge packed with gel particles or silica gel particles or the like. A method for improving resolution of the column (cartridge) having generally been adopted is either one of increase in packing density of the solid phase material, and reduction in number-average diameter of the filler. However, the former has failed in increasing the packing density up to an expected level, due to variation in geometry and diameter of the filler, whereas the latter has tended to be limited in process speed, due to increased load of pressure to the column or apparatus. In addition, a small-scale column (cartridge) has been more likely to degrade resolution and yield, due to a dead volume of a frit (a component for stopping beads) necessary for holding the filler.

The present invention now adopts monolithic silica as a means for avoiding these problems. Monolithic silica is referred to as a silica given in a form of porous continuous article having a three-dimensional network structure, and has advantages over the conventional granular silica in terms of good liquid permeability, frit-less configuration, and small dead volume (Japanese Laid-Open Patent Publication No. H06-265534). Monolithic silica is preferably immobilized in a columnar vessel, or to a multi-well plate.

Too small pore size of monolithic silica may degrade the liquid permeability, whereas too large pore size may reduce theoretical plate number to thereby reduce yield of sugar chain. For this reason, the pore size of monolithic silica is preferably 1 to 100 µm in terms of diameter of through-pores communicated with each other, more preferably 1 to 50 µm, still more preferably 1 to 30 µm, and most preferably 1 to 20 µm.
Preferable mode of use of monolithic silica may be normal phase, or HILIC (Hydrophilic interaction chromatography).

Monolithic silica is preferably used in such a way that the sample solution containing a labeled sugar chain is injected into a column or multi-well plate having monolithic silica immobilized therein, then allowed to pass through the monolithic silica portion based on free fall, suction, pressurization, or centrifugation, and washed with a washing liquid, and added with an eluant to thereby elute the sugar chain.

The sample solution to be applied to monolithic silica preferably contains 90% by volume or more of an organic solvent, and more preferably contains 95% by volume or more of an organic solvent. The organic solvent adoptable herein includes acetonitrile, methanol, ethanol, 2-propanol, hexane, ethyl acetate, methylene chloride, and tetrahydrofuran, wherein acetonitrile is most preferable.

Monolithic silica, after being applied with the sugar chain sample, is preferably washed with a washing liquid. The washing liquid preferably contains 90% by volume or more of an organic solvent and 10% by volume or less of water, and more preferably contains 95% by volume or more of an organic solvent and 5% by volume or less of water. The organic solvent adoptable herein include acetonitrile, methanol, ethanol, 2-propanol, hexane, ethyl acetate, methylene chloride, and tetrahydrofuran, wherein acetonitrile is most preferable. It is also preferable to sequentially wash monolithic silica, using washing liquids having different compositions, such as 100% acetonitrile and an acetonitrile/water (95:5,v/v) mixed solvent.

After the washing, monolithic silica is preferably added with an eluant to thereby elute the absorbed labeled sugar chain. The eluant preferably contains 10% by volume or more of water, more preferably contains 50% by volume or more of water, and most preferably nothing but water. Components other than water is preferably an organic solvent selected from acetonitrile, and alcohols represented by methanol, ethanol, propanol, and butanol.
The collected sugar chain solution may be subjected to various measurements, after being concentrated, dried or diluted if occasions demand.

### (Analysis of Sugar Chain Using MALDI-TOF MS)

The thus-obtained labeled sugar chain may be analyzed by mass spectrometry represented by MALDI-TOF MS. In particular, for the case where the sugar chain is labeled with N-aminooxyacetyl-tryptophanyl (arginine methyl ester), MALDI-TOF MS may ensure high-sensitivity analysis.

### (Analysis of Sugar Chain Using HPLC)

The thus-obtained labeled sugar chain may be analyzed by HPLC. In particular, the sugar chain of PA-modified, 2AB-modified, 2AA-modified, or AA-Ac-modified species is analyzable in a particularly preferable manner.

### (Other Analyses of Sugar Chain)

The thus-obtained labeled sugar chain may be subjected to various analyses including LC-MS, HPLC-MS, capillary electrophoresis and so forth.

### EXAMPLES

The present invention will specifically be explained in Examples below, without limiting the invention to these Examples.

### (Preparation of Sugar Chain Sample)

Fetuin (derived from fetal bovine serum) was used as a model glycoprotein. One milligram of fetuin was placed in a vessel, and dissolved into 50 µL of a 100 mM ammonium bicarbonate solution. The solution was added with 5 µL of a 120 mM dithiothreitol solution, incubated at 60°C for 30 minutes, added with 10 µL of a 123 mM iodoacetamide solution, and allowed to stand at room temperature for 1 hour. The mixture was further added with added with 400 unit of trypsin, and allowed to stand at 37°C for 16 hours. The mixture was then treated at 90°C for 5 minutes to inactivate trypsin, added with 1 unit of N-glycosidase F (from Roche Diagnostics), and incubated at 37°C for 16 hours to release the sugar chain. The mixture was added with pure water to adjust the total volume to 100 µL.

### (Purification of Sugar Chain)

The sugar chain was purified using a polymer bead capable of scavenging sugar chain. Twenty microliters of the thus-obtained fetuin sugar chain solution (equivalent to 200 µg of fetuin) was added to 5 mg of hydrazide group-containing polymer bead (from Sumitomo Bakelite Co., Ltd., BS-45601S), added with 180 µL of acetonitrile containing 2% acetic acid, the mixture was allowed to react at 80°C for 1 hour, and then dried. The polymer bead was washed with a 2 M guanidine hydrochloride solution, water, methanol and 1% triethylamine solution, then added with a 10% acetic anhydride/methanol solution, and allowed to react at room temperature for 30 minutes to thereby cap the hydrazide group. After the capping, the polymer bead was washed with methanol and water. The bead was added with 20 µL of pure water and 180 µL of acetonitrile containing 2% acetic acid, and dried under heating at 80°C for 1 hour to thereby release therefrom the sugar chain.

### (Labeling of Sugar Chain)

The sugar chain was labeled by either one of two methods described below.

### (Labeling with 2AB)

2-Aminobenzamide was dissolved into a 30%(v/v) acetic acid/DMSO mixed solution so as to adjust the final concentration to 350 mM. The solution was further added with sodium cyanoborohydride as a reducing agent, so as to adjust the final concentration to 1 M. Fifty microliters of this solution was added to the above-described bead, and allowed to react at 80°C for 2 hours, to thereby label the sugar chain with 2AB. The supernatant is collected to thereby obtain a 2AB-labeled sugar chain solution (containing unreacted 2AB).

### (Labeling with PA)

Dissolved was 552 mg of 2-aminopyridine into 200 µg of acetic acid (PA solution). Two hundred milligrams of dimethylamine-borane was dissolved into an aqueous acetic acid solution (mixture of 50 µL of pure water and 80 µL of acetic acid) (reducing agent solution).
Thirty microliters of the PA solution was added to the above-described bead, and allowed to react at 90°C for 1 hour. Thereafter, the bead was added with 110 µL of the reducing agent solution, and allowed to react at 80°C for 35 minutes.

### (Purification of Labeled Sugar Chain)

Solid phase extraction was carried out in order to remove the unreacted labeling reagent.

### (Example 1)

The unreacted 2AB was removed from the 2AB-labeled sample solution, using monolithic silica. Monolithic silica adopted herein was "MonoFas spin column" from GL Sciences Inc. The column has a discotic monolithic silica filter with a through-pore diameter of 15 µm, fixed to the bottom of a tube having a capacity of approximately 1 mL, and is designed to allow a sample solution, injected into the tube, to pass through the pores of monolithic silica under centrifugation.
The thus-obtained 2AB-labeled sugar chain solution was diluted with acetonitrile, so as to adjust the acetonitrile content to 95% (v/v). The whole volume of the sample solution was injected into the monolithic silica spin column, and allowed to pass therethrough under centrifugation using a desktop centrifugal separator, so as to make it adsorb the labeled sugar chain contained in the sample. The column was sequentially washed with acetonitrile, and acetonitrile/water (95:5, v/v), and the adsorbed component was eluted using 50 µL or pure water, and the eluate was collected.

### (Example 2)

Unreacted PA was removed from a PA-labeled sample solution using monolithic silica. Procedures were same as those in Example 1.

### (Comparative Example 1)

The unreacted 2AB was removed from the 2AB-labeled sample solution using a granular silica gel. The silica gel adopted herein was "Iatrobeads 6RS-8060" from Mitsubishi Kagaku Iatron, Inc. Approximately 30 mg of silica gel was packed into a disposable column (a polypropylene column attached with a polyethylene frit), the sample solution prepared similarly as described in Example 1 was placed thereon, and was allowed to cause free fall in the column to thereby make the silica gel adsorb the labeled sugar chain contained in the sample. The column was then sequentially washed with acetonitrile, and acetonitrile /water (95:5, v/v), the adsorbed component was eluted using 50 µL of pure water, and the eluate was collected.

### (HPLC Measurement)

Each of the sugar chain solutions obtained in the Examples and Comparative Example was measured by HPLC. An HPLC apparatus adopted herein was LaChrom ELITE from Hitachi High-Technologies Corporation (with L-2130 pump, L-2200 auto-sampler, L-2350 column oven, L-2485 phosphorescence detector), and an analytical column adopted herein was Shodex Asahipak NH2P-50 4E from Showa Denko K.K. The analysis was carried out based on a gradient mode, using (A) 2%(v/v) acetic acid /acetonitrile solution, and (B) 3%(v/v) acetic acid, 5%(v/v) triethylamine/water solution as the mobile phase, while varying the ratio of (A) from 70% to 5% over 90 minutes. Column temperature was kept at 40°C. Detection was based on phosphorescence, while being excited at 330 nm and detected at a phosphorescence wavelength of 420 nm in Example 1 and Comparative Example (2AB-modified sugar chains), whereas excited at 320 nm and detected at a phosphorescence wavelength of 400 nm in Example 2 (PA-modified sugar chain). Amount of injection of the sample solution was adjusted to 1/20 of the whole sample solution, which is equivalent to 10 µg of fetuin.

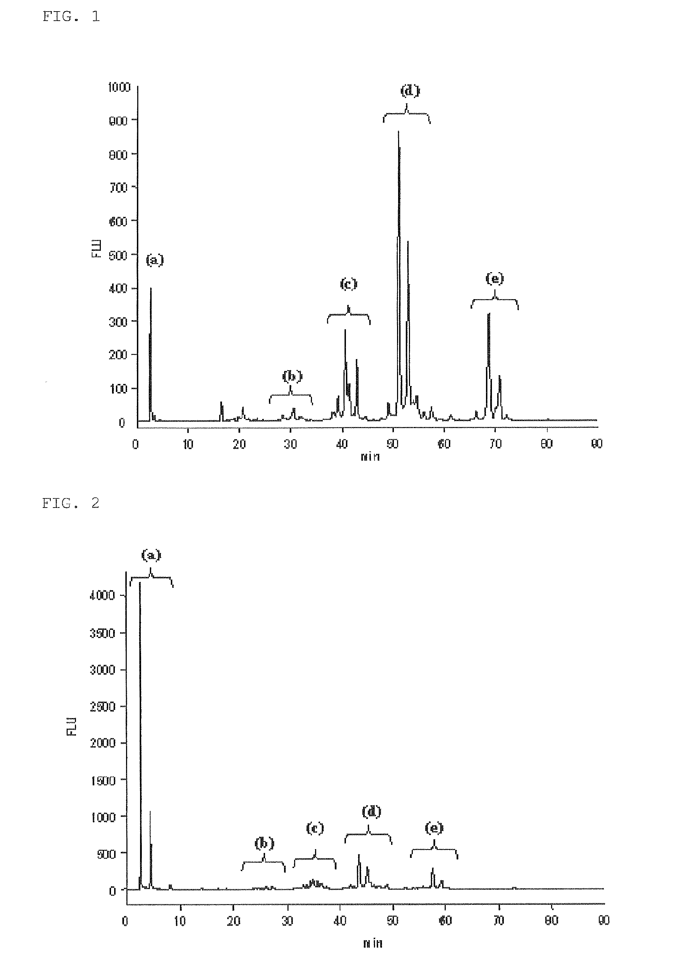

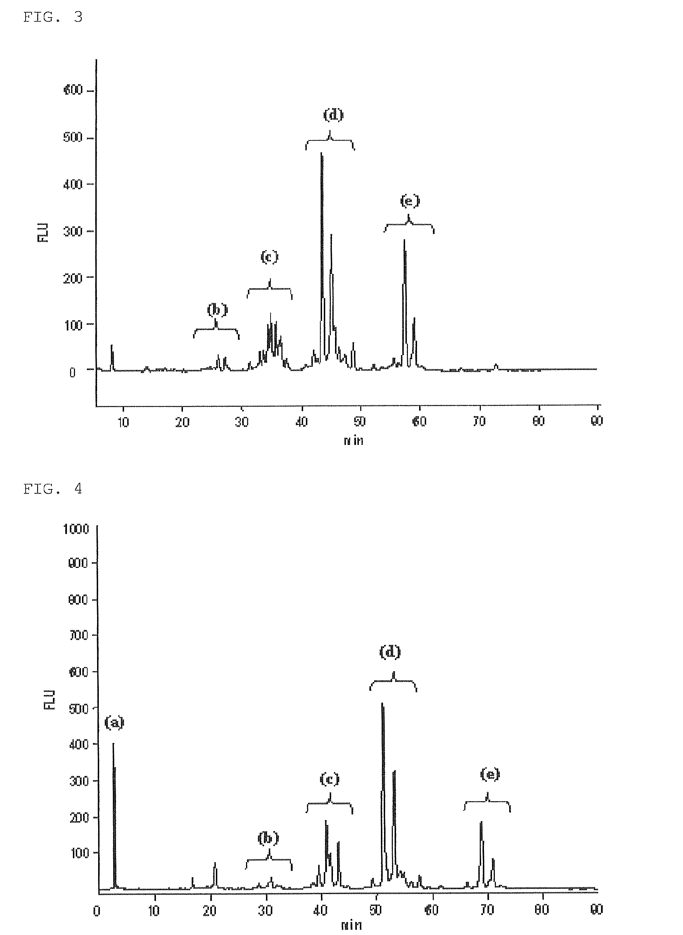

FIG. 1 illustrates an HPLC chart of the 2AB-modified sugar chain prepared by the method of Example 1 (monolithic silica). FIG. 2 illustrates an HPLC chart of the PA-modified sugar chain prepared by the method of Example 2 (monolithic silica), and FIG. 3 illustrates an enlarged view of FIG. 2. FIG. 4 illustrates an HPLC chart of 2AB-modified sugar chain prepared by the method of Comparative Example (granular silica gel).
The abscissa represents retention time (min.), and the ordinate represents phosphorescence intensity (a.u.). In the charts, (a) represents peak (s) assignable to the unreacted labeling reagent, (b) represents peaks assignable to a sugar chain containing a single sialic acid residue, (c) represents peaks assignable to a sugar chain containing two sialic acid residues, (d) represents peaks assignable to a sugar chain containing three sialic acid residues, and (e) represents peaks assignable to a sugar chain containing four sialic acid residues.
Table 1 shows areas of the individual peaks (a) to (e) in the HPLC charts obtained in Example 1 and Comparative Example 1.

**[Table 1]**

| | Peak | Example 1 | Peak area Comparative Example 1 |
|---|---|---|---|
| (a) | Unreacted 2AB | 13182461 | 13729872 |
| (b) | Sialic acid ×1 | 11398824 | 9497382 |
| (c) | Sialic acid ×2 | 76812951 | 58779242 |
| (d) | Sialic acid ×3 | 202539136 | 125811902 |
| (e) | Sialic acid ×4 | 74898917 | 43519005 |

In both of Example and Comparative Example, it was proved that the peak (s) assignable to the unreacted labeling reagent ((a) in the charts) was small enough so as not to inhibit detection of the peaks assignable to the sugar chains((b) to (e) in the charts), and that both methods were successful to thoroughly remove the unreacted reagents. Referring now to Example 1 and Comparative Example 1, it was found that the areas of the peaks assignable to the 2AB-modified fetuin sugar chains ((b) to (e) in the charts) were explicitly larger in Example 1 than in Comparative Example 1, proving that the use of monolithic silica resulted in a larger yield of the labeled sugar chain. In both methods, patterns of the peaks were found to be in good agreement with the pattern reported in literatures (J. Proteome Res., 4, pp.146-152(2005), and so forth).
The monolithic silica adopted in the present invention has been adopted typically to purification of nucleic acids (International Publication Pamphlet WO2005-078088, Japanese Laid-Open Patent Publication No. 2006-296220). On the other hand, granular silica, graphite carbon, beads for gel filtration and so forth have conventionally been used for purification of the labeled sugar chains. In the present invention, the inventors made a trial on applying monolithic silica to separation of the labeled sugar chains and the unreacted labeling reagent. As a consequence, by using monolithic silica, procedures for purifying the sugar chains were simplified as compared with those in the conventional methods, and efficiency of purification of sugar chain was successfully improved.

### INDUSTRIAL APPLICABILITY

Use of the method of the present invention successfully simplifies the removal process of the unreacted reagent after the labeling reaction of the sugar chains, and improves the yield, so that the present invention is applicable not only to studies on sugar chains, but also to recovery of sugar chains from natural products, manufacturing of standard substance of sugar chain, and still also to the field of medical science including exploration of disease marker based on sugar chain analysis, and diagnosis based on sugar chain analysis.

## Claims

1. A method of preparing a sugar chain sample for reducing unreacted labeling reagent in a sample solution containing a labeled sugar chain, comprising:
(process 1) a process of bringing the sample solution containing the labeled sugar chain into contact with monolithic silica, so as to allow said monolithic silica to adsorb a sugar chain component;
(process 2) a process of washing said monolithic silica with a washing liquid; and
(process 3) a process of bringing said monolithic silica into contact with an eluant, so as to elute the adsorbed sugar chain.

2. The method of preparing a sugar chain sample as claimed in Claim 1, wherein process 1 includes a process of injecting sample solution into a columnar vessel having monolithic silica packed in the flow path thereof; and a process of allowing said sample solution to pass through the monolithic silica portion based on free fall, suction, pressurization or centrifugation.

3. The method of preparing a sugar chain sample as claimed in Claim 1 or 2, wherein, in process 1, said sample solution contains 90% by volume or more of an organic solvent.

4. The method of preparing a sugar chain sample as claimed in Claim 1 or 2, wherein, in process 1, said sample solution contains 95% by volume or more of an organic solvent.

5. The method of preparing a sugar chain sample as claimed in Claim 3 or 4, wherein said organic solvent is acetonitrile.

6. The method of preparing a sugar chain sample as claimed in any one of Claims 1 to 5, wherein, in process 2, said washing liquid is a solution containing an organic solvent and water.

7. The method of preparing a sugar chain sample as claimed in Claim 6, wherein, in process 2, said washing liquid is a solution containing 90% by volume or more of said organic solvent and 10% by volume or less of water.

8. The method of preparing a sugar chain sample as claimed in Claim 6, wherein, in process 2, said washing liquid contains 95% by volume or more of said organic solvent, and 5% by volume or less of water.

9. The method of preparing a sugar chain sample as claimed in any one of Claims 6 to 8, wherein said organic solvent is acetonitrile.

10. The method of preparing a sugar chain sample as claimed in any one of Claims 1 to 9, wherein, in process 3, said eluant is a solution containing 10% by volume or more of water.

11. The method of preparing a sugar chain sample as claimed in any one of Claims 1 to 9, wherein, in process 3, said eluant is a solution containing 50% by volume or more of water.

12. The method of preparing a sugar chain sample as claimed in any one of Claims 1 to 9, wherein, in process 3, said eluant is water.

13. The method of preparing a sugar chain sample as claimed in any one of Claims 1 to 12, wherein said labeled sugar chain has a compound having an amino group, hydrazide group, or aminoxy group, bound to the reducing end thereof.

14. The method of preparing a sugar chain sample as claimed in any one of Claims 1 to 12, wherein said labeled sugar chain has at least one amino group-containing compound selected from those listed below, bound to the reducing end thereof:
2-aminopyridine; 2-aminobenzamide; 2-aminoanthranilic acid;
7-amino-1-naphthol; 3-(acetylamino)-6-aminoacridine;
9-aminopyrene-1,4,6-trisulfonic acid;
8-aminonaphtalene-1,3,6-trisulfonic acid;
7--amino-1,3-naphtalenedisulfonic acid: 2-amino-9(10H)-acridone;
5-aminofluorescein; Dansylethylenediamine;
7-amino-4-methylcoumarine; and benzylamine.

15. The method of preparing a sugar chain sample as claimed in any one of Claims 1 to 12, wherein said labeled sugar chain has at least one hydrazide group-containing compound selected from those listed below, bound to the reducing end thereof:
2-aminobenzhydrazide; 2-hydrazinobenzoic acid; benzylhydrazine;
5-Dimethylaminonaphthalene-1-sulfonyl hydrazine (Dansylhydrazine); 2-hydrazinopyridine; 9-fluorenylmethyl carbazate (Fmoc hydrazine);
4,4-difluoro-5,7-dimethyl-4-bora-3a,4a-diaza-s-indacene-3-propionic acid, hydrazide;
2-(6,8-difluoro-7-hydroxy-4-methylcoumarin) acetohydrazide;
7-diethylaminocoumarin-3-carboxylic acid, hydrazide (DCCH); phenylhydrazine;1-Naphthaleneacethydrazide; and phenylacetic hydrazide.

16. The method of preparing a sugar chain sample as claimed in any one of Claims 1 to 12, wherein said labeled sugar chain has at least one aminoxy group-containing compound selected from those listed below, bound to the reducing end thereof:
N-aminooxyacetyl(tryptophyl)arginine methyl ester;
O-benzylhydroxylamine; O-phenylhydroxylamine; O-(2,3,4,5,6-pentafluorobenzyl)hydroxylamine; 0-(4-nitrobenzyl)hydroxylamine;
2- aminooxypyridine; 2-aminooxymethylpyridine; 4-[(aminooxyacetyl) amino]benzoic acid methyl ester; 4-[(aminooxyacetyl)amino]benzoic acid ethyl ester; 4-[(aminooxyacetyl)amino]benzoic acid *n*-butyl ester; and aminooxy-biotin.

17. A sugar chain sample prepared by the method of preparing a sugar chain sample described in any one of Claims 1 to 16.

18. A method of analyzing a sugar chain analyzing said sugar chain sample, prepared by the method of preparing a sugar chain sample described in any one of Claims 1 to 16, by an analytical means such as HPLC, mass spectrometry, LC-MS, or capillary electrophoresis.
